(19) Europäisches Patentamt / European Patent Office / Office européen des brevets

(11) **EP 2 247 275 B1**

(12) **EUROPEAN PATENT SPECIFICATION**

(45) Date of publication and mention
of the grant of the patent:
**29.11.2017 Bulletin 2017/48**

(21) Application number: **09711341.9**

(22) Date of filing: **12.02.2009**

(51) Int Cl.:
*A61K 8/04* *(2006.01)*          *A61K 8/11* *(2006.01)*
*A61K 8/19* *(2006.01)*          *A61K 8/23* *(2006.01)*
*A61K 8/25* *(2006.01)*          *A61K 8/26* *(2006.01)*
*A61K 8/31* *(2006.01)*          *A61K 8/34* *(2006.01)*
*A61K 8/36* *(2006.01)*          *A61K 8/73* *(2006.01)*
*A61K 8/81* *(2006.01)*          *A61K 8/84* *(2006.01)*
*A61K 8/86* *(2006.01)*          *A61K 8/92* *(2006.01)*
*A61Q 19/00* *(2006.01)*

(86) International application number:
**PCT/IB2009/050576**

(87) International publication number:
**WO 2009/101593 (20.08.2009 Gazette 2009/34)**

(54) **DELIVERY PARTICLE**

FREISETZUNGSPARTIKEL

PARTICULE D'ADMINISTRATION

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **15.02.2008 US 65906 P**

(43) Date of publication of application:
**10.11.2010 Bulletin 2010/45**

(73) Proprietor: **The Procter & Gamble Company
Cincinnati, OH 45202 (US)**

(72) Inventors:
• **SOMERVILLE ROBERTS, Nigel, Patrick
Newcastle Upon Tyne
Northumberland NE20 9UJ (GB)**
• **GUILLARD, Nicolas
Newcastle Upon Tyne
Tyne And Wear NE7 7RG (GB)**
• **MARTIN DE JUAN, Luis
Whitley Bay
Tyne And Wear NE262NL (GB)**
• **SMETS, Johan
B-3210 Lubbeek (BE)**
• **BURDIS, John, Allen
Newcastle Upon Tyne
Tyne And Wear NE5 2QU (GB)**
• **LAW, Daniel, Ning Geng
Beijing 100085 (CN)**

(74) Representative: **Pickford, James Lawrence
Procter & Gamble
Technical Centres Limited
Whitley Road
Longbenton
Newcastle upon Tyne NE12 9TS (GB)**

(56) References cited:
**EP-A- 0 238 225          EP-A- 0 393 289
WO-A-2006/056093          WO-A-2006/131846
WO-A-2007/099469**

**Description**

FIELD OF THE INVENTION

The present application relates to agglomerates/particles comprising encapsulated, benefit agents, compositions comprising such agglomerates/particles, and processes for making and using such agglomerates/particles and compositions comprising such agglomerates/particles.

BACKGROUND OF THE INVENTION

Benefit agents, such as perfumes, silicones, waxes, flavors, vitamins and fabric softening agents, are expensive and generally less effective when employed at high levels in personal care compositions, cleaning compositions, and fabric care compositions. As a result, there is a desire to maximize the effectiveness of such benefit agents. One method of achieving this objective is to improve the delivery efficiencies of such benefit agents. Unfortunately, it is difficult to improve the delivery efficiencies of benefit agents as such agents may be lost do to the agents' physical or chemical characteristics, or such agents may be incompatible with other compositional components or the situs that is treated.

In an effort to improve the delivery efficiencies of benefit agents, the industry, in many cases, encapsulated such benefit agents. WO2007/099469, WO2006/131846 and EP-A-383289 relate to methods of encapsulating benefit agents. WO2006/056093 relates to encapsulated benefit agents made into a slurry to provide agglomerated encapsulated benefit agents. EP-A-238225 relates to encapsulated colorant for use in cosmetic formulations. Unfortunately, when an encapsulate is incorporated into a dry product, the encapsulated benefit agent is typically damaged and thus prematurely releases the benefit agent.

Accordingly, there is a need for a process and a benefit delivery agent that minimizes or eliminates such drawback.

SUMMARY OF THE INVENTION

The present application relates to a process of producing a cleaning composition comprising a benefit agent delivery composition in the form of an agglomerate, particulate or extrudate and an adjunct ingredient, said process comprising:

a) combining (i) an encapsulated benefit agent, wherein said encapsulated benefit agent comprises a perfume microcapsule, said perfume microcapsule comprising a shell, said shell comprising cross-linked melamine formaldehyde (ii) a plasticizer, wherein said plasticizer comprises water and iii) a binder, wherein said binder is selected from the group consisting of celluloses preferably methylcellulose, more preferably CMC, and derivatives thereof; alginate and derivatives thereof; starches; polyvinyl alcohols; polyethylene oxide; polyvinylpyrolidone; polysaccharides preferably chitosan and/or natural gums preferably carrageenan; polyacrylates preferably cross-linked polyacrylates; waxes; polyethylene glycols having a weight average molecular weight of greater than 4000 Da or even from 4000 Da to 15,000 Da; alcohol ethoxylates; surfactants and mixtures thereofto form a mixture;

b) combining said mixture with a dusting agent, wherein said dusting agent comprises a material selected from the group consisting of silicas; aluminosilicates preferably zeolite; clays; and mixtures thereof to form a material; and

c) removing a sufficient amount of said plasticizer from said material to yield a benefit agent delivery composition in the form of an agglomerate, particulate or extrudate comprising, based on total weight, delivery composition from 2% to 97% by weight encapsulated benefit agent comprising a sufficient amount of benefit agent to provide, based on total weight of benefit delivery composition, from 1 % to 85% benefit agent, from 1 % to 50% by weight, preferably from 2% to 45% by weight plasticizer, from 1 to 50 wt% binder and from 5%-50% by weight dusting agent; and

d) mixing with an adjunct ingredient to form a cleaning composition.

DETAILED DESCRIPTION OF THE INVENTION

<u>Definitions</u>

As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists.

As used herein, the term "fabric care composition" includes, unless otherwise indicated, fabric softening compositions, fabric enhancing compositions, fabric freshening compositions and combinations there of.

As used herein, the terms "particle", "benefit agent containing delivery particle", are synonymous, and the terms "capsule" and "microcapsule" are synonymous.

As used herein, the articles including "a" and "an" when used in a claim, are understood to mean one or more of what is claimed or described.

As used herein, the terms "include", "includes" and "including" are meant to be non-limiting.

The test methods disclosed in the Test Methods Section of the present application may be used to determine the respective values of the parameters of Applicants' inventions.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Benefit Agent Delivery Composition - Agglomerate/Particle/Extrudate

In one aspect, a benefit agent delivery composition that may be an agglomerate, particulate or extrudate that may comprise, based on total benefit agent delivery weight:

    a.) from about 2% to about 97%, from about 10% to about 90%, from about 15% to about 85%, from about 20 % to about 80%, from about 25% to about 75%, or even from about 30% to about 70%.of an encapsulated benefit agent comprising a sufficient amount of benefit agent to provide, based on total benefit delivery composition weight from about 1% to about 85%, from about 8% to about 80%, from about 12% to about 75%, from about 15% to about 65%, from about 20% to about 60%, or even from about 25% to about 55% benefit agent;

    b.) from about 1% to about 50%, from about 2% to about 45%, from about 3% to about 40%, from about 4% to about 37%, from about 5% to about 35%, or even from about 6% to about 30% of a plasticizer;

    c.) from about 1% to about 50%, from about 2% to about 45%, from about 3% to about 35%, from about 4% to about 30%, from about 5% to about 25%, or even from about 6% to about 20% of a binder; and

d.) optionally, from about 1% to about 50%, from about 2% to about 45%, from about 5% to about 40%, from about 7% to about 35%, from about 9% to about 30%, or even from about 10% to about 27% of a dusting agent is disclosed.

In one aspect of the aforementioned benefit agent delivery composition said plasticizer may comprise a material selected from the group consisting of water; alcohols including glycerol, ethanol and/or propan-1-diol; glycols including polyethylene glycols, for example, polyethylene glycols having a molecular weight (weight average) of less than 600 Da or even from about 600 Da to about 200 Da; fatty acids; petroleum derivatives including paraffins, petrolatum and/or mineral oils; vegetable oils; and mixtures thereof; in one aspect, said plasticizer may comprise a material selected from the group consisting of water and alcohols and mixtures thereof; in one aspect, said plasticizer may comprise a material selected from the group consisting of water and glycerol and mixtures thereof; in one aspect, said plasticizer comprises water.

In one aspect of the aforementioned benefit agent delivery composition said binder may comprise a material selected from the group consisting of celluloses including methylcellulose, including CMC, and derivatives thereof; alginate and derivatives thereof; starches; polyvinyl alcohols; polyethylene oxide; polyvinylpyrolidone; polysaccharides including chitosan and/or natural gums including carrageenan; polyacrylates including cross-linked polyacrylates; waxes; polyethylene glycols for example, polyethylene glycols having a molecular weight (weight average) of greater than 4000 Da or even from about 4000 Da to about 15,000 Da; alcohol ethoxylates; surfactants and mixtures thereof; in one aspect, said binder may comprise a material selected from the group consisting of celluloses including methylcellulose, including CMC, and derivatives thereof; alginate and derivatives thereof; starches; polyvinyl alcohols; polysaccharides including chitosan and/or natural gums including carrageenan; polyacrylates including cross-linked polyacrylates; polyethylene glycols for example, polyethylene glycols having a molecular weight (weight average) of greater than 4000 Da or even from about 4000 Da to about 15,000 Da; and mixtures thereof; in one aspect said binder may comprise a material selected from the group consisting of celluloses including methylcellulose, including CMC, and derivatives thereof; alginate and derivatives thereof; starches; polyvinyl alcohols; polyacrylates including cross-linked polyacrylates; polyethylene glycols for example, polyethylene glycols having a molecular weight (weight average) of greater than 4000 Da or even from about 4000 Da to about 15,000 Da; and mixtures thereof; in one aspect said binder may comprise a material selected from the group consisting of celluloses including methylcellulose, including CMC; polyacrylates including cross-linked polyacrylates; polyethylene glycols for example, polyethylene glycols having a molecular weight (weight average) of greater than 4000 Da or even from about 4000 Da to about 15,000 Da; and mixtures thereof.

In one aspect of the aforementioned benefit agent delivery composition, said dusting agent may comprise a material selected from the group consisting of silicas; zeolites; amorphous aluminosilicates; clays; starches; celluloses; water soluble salts including sodium chloride, sodium sulphate, magnesium sulphate and/or sodium carbonate; polysaccharides including sugars; and mixtures thereof; in one aspect dusting agent may comprise a material selected from the group consisting of silicas; aluminosilicates including zeolite; clays; starches; celluloses; polysaccharides including sugars; and mixtures thereof; in one aspect said dusting agent may comprise a material selected from the group consisting of silicas; aluminosilicates including zeolite; clays; starches; celluloses; and mixtures thereof; in one aspect said dusting agent may comprise a material selected from the group consisting of silicas; aluminosilicates including zeolite; clays; and mixtures thereof.

In one aspect of the aforementioned benefit agent delivery composition, said encapsulated benefit agent may comprise a perfume microcapsule, a binder and mixtures thereof. In one aspect, the aforementioned benefit agent delivery composition said perfume microcapsule comprises a shell, said shell comprising cross-linked melamine formaldehyde.

In one aspect of the aforementioned benefit agent delivery composition

a. said encapsulated benefit agent may comprise a perfume microcapsule, said perfume microcapsule comprising a shell, said shell comprising cross-linked melamine formaldehyde;
b. said plasticizer may comprise water;
c. said binder may be selected from the group consisting of celluloses including methylcellulose, including CMC, and derivatives thereof; alginate and derivatives thereof; starches; polyvinyl alcohols; polyethylene oxide; polyvinylpyrolidone; polysaccharides including chitosan and/or natural gums including carrageenan; polyacrylates including cross-linked polyacrylates; waxes; polyethylene glycols for example, polyethylene glycols having a molecular weight (weight average) of greater than 4000 Da or even from about 4000 Da to about 15,000 Da; alcohol ethoxylates; surfactants and mixtures thereof; and
d. said dusting agent may be selected from the group consisting of silicas; zeolites; amorphous aluminosilicates; clays; starches; celluloses; water soluble salts including sodium chloride, sodium sulphate, magnesium sulphate and/or sodium carbonate; polysaccharides including sugars; and mixtures thereof.

In one aspect of the aforementioned benefit agent delivery composition, said agglomerate, extrudate or particulate may have a characteristic dimension of about 100 microns to about 3000 microns, from about 200 microns to about 2500 microns, from about 300 microns to about 2000 microns, from about 400 microns to about 1400 microns, or even from about 500 microns to about 1200 microns, wherein for said agglomerates and particulates said characteristic dimension is the median particle size of said agglomerates and particulates and the characteristic dimension of said extrudates is the mean diameter of said extrudates.

Benefit Agent Delivery Composition - Agglomerate/Particle/Extrudate

The benefit agent delivery compositions of the present invention may be made in accordance with the examples of the present specification and/or by the following process which may comprise:

a.) combining an encapsulate, a plasticizer and a binder to form a mixture;
b.) combining said mixture with said dusting agent to form a material; and
c.) removing a sufficient amount of said plasticizer from said material to yield a product comprising, based on total product weight from about 1% to about 50%, from about 2% to about 45%, from about 3% to about 40%, from about 4% to about 37%, from about 5% to about 35%, or even from about 6% to about 30% plasticizer.

Benefit Agent Containing Delivery Particle

Applicants discovered that the problems associated of incorporating an encapsulated benefit agent into a dry product, including the premature rupturing of the shell of the encapsulated during the incorporation process, can be minimized when the encapsulated benefit agent is further processed and incorporated into an agglomerate that can then be added to a consumer product such as a dry consumer product that may be a particulate, powder or other essentially dry form.

The wall materials of useful encapsulates may comprise materials selected from the group consisting of polyethylenes, polyamides, polystyrenes, polyisoprenes, polycarbonates, polyesters, polyacrylates, polyureas, polyurethanes, polyolefins, polysaccharides, epoxy resins, vinyl polymers, and mixtures thereof. In one aspect, useful wall materials include materials that are sufficiently impervious to the core material and the materials in the environment in which the encapsulated benefit agent will be employed, to permit the delivery benefit to be obtained. Suitable impervious wall materials include materials selected from the group consisting of reaction products of one or more amines with one or more aldehydes, such as urea cross-linked with formaldehyde or gluteraldehyde, melamine cross-linked with formaldehyde; gelatin-polyphosphate coacervates optionally cross-linked with gluteraldehyde; gelatin-gum Arabic coacervates; cross-linked silicone fluids; polyamine reacted with polyisocyanates and mixtures thereof. In one aspect, the wall material comprises melamine cross-linked with formaldehyde.

The core of the encapsulated benefit agent may comprise perfume raw materials, silicone oils, waxes, hydrocarbons, higher fatty acids, essential oils, lipids, skin coolants, vitamins, sunscreens, antioxidants, glycerine, catalysts, bleach particles, silicon dioxide particles, malodor reducing agents, odor-controlling materials, chelating agents, antistatic agents, softening agents, insect and moth repelling agents, colorants, antioxidants, chelants, bodying agents, drape and form control agents, smoothness agents, wrinkle control agents, sanitization agents, disinfecting agents, germ control agents, mold control agents, mildew control agents, antiviral agents, drying agents, stain resistance agents, soil release agents, fabric refreshing agents and freshness extending agents, chlorine bleach odor control agents, dye fixatives, dye transfer inhibitors, color maintenance agents, optical brighteners, color restoration/rejuvenation agents, anti-fading agents, whiteness enhancers, anti-abrasion agents, wear resistance agents, fabric integrity agents, antiwear agents, anti-pilling agents, defoamers and anti-foaming agents, UV protection agents for fabrics and skin, sun fade inhibitors, anti-allergenic agents, enzymes, water proofing agents, fabric comfort agents, shrinkage resistance agents, stretch resistance agents, stretch recovery agents, skin care agents, glycerin, and natural actives such as aloe vera, vitamin E, shea butter, cocoa butter, and the like, brighteners, antibacterial actives, antiperspirant actives, cationic polymers and mixtures thereof. In one aspect, said perfume raw material is selected from the group consisting of alcohols, ketones, aldehydes, esters, ethers, nitriles alkenes. In one aspect the core material may comprise a perfume. In one aspect, said perfume may comprise perfume raw materials selected from the group consisting of alcohols, ketones, aldehydes, esters, ethers, nitriles alkenes and mixtures thereof. In one aspect, said perfume may comprise a perfume raw material selected from the group consisting of perfume raw materials having a boiling point (B.P.) lower than about 250°C and a ClogP lower than about 3, perfume raw materials having a B.P. of greater than about 250°C and a ClogP of greater than about 3, perfume raw materials having a B.P. of greater than about 250°C and a ClogP lower than about 3, perfume raw materials having a B.P. lower than about 250°C and a ClogP greater than about 3 and mixtures thereof. Perfume raw materials having a boiling point B.P. lower than about 250°C and a ClogP lower than about 3 are known as Quadrant I perfume raw materials, perfume raw materials having a B.P. of greater than about 250°C and a ClogP of greater than about 3 are known as Quadrant IV perfume raw materials, perfume raw materials having a B.P. of greater than about 250°C and a ClogP lower than about 3 are known as Quadrant II perfume raw materials, perfume raw materials having a B.P. lower than about 250°C and a ClogP greater than about 3 are known as a Quadrant III perfume raw materials. In one aspect, said perfume comprises a perfume raw material having B.P. of lower than about 250°C. In one aspect, said perfume may comprise a perfume raw material selected from the group consisting of Quadrant I, II, III perfume raw materials and mixtures thereof. In one aspect, said perfume comprises a Quadrant III perfume raw material. Suitable Quadrant I, II, III and IV perfume raw materials are disclosed in U.S. patent 6,869,923 B1.

In one aspect, said perfume may comprise a Quadrant IV perfume raw material. While not being bound by theory, it is believed that such Quadrant IV perfume raw materials can improve perfume odor "balance". Said perfume may comprise, based on total perfume weight, less than about 30%, less than about 20%, or even less than about 15% of said Quadrant IV perfume raw material.

The perfume raw materials and accords may be obtained from one or more of the following companies Firmenich (Geneva, Switzerland), Givaudan (Argenteuil, France), IFF (Hazlet, NJ), Quest (Mount Olive, NJ), Bedoukian (Danbury, CT), Sigma Aldrich (St. Louis, MO), Millennium Specialty Chemicals (Olympia Fields, IL), Polarone International (Jersey City, NJ), Fragrance Resources (Keyport, NJ), and Aroma & Flavor Specialties (Danbury, CT).

Process of Making Encapsulated Benefit Agents

The encapsulated benefit agents employed herein may be made via the teachings of USP 6,592,990 B2 and/or USP 6,544,926 B1 and the examples disclosed herein.

Anionic emulsifiers are typically used during the encapsulation process to emulsify the benefit agent prior to microcapsule formation. While not being bound by theory, it is believed that the anionic materials adversely interact with the cationic surfactant actives that are often found in compositions such as fabric care compositions - this may yield an aesthetically unpleasing aggregation of particles that are employed in said composition. In addition to the unacceptable aesthetics, such aggregates may result in rapid phase separation of the particles from the bulk phase. Applicants discovered that such aggregates can be prevented by the addition of certain aggregate inhibiting materials including materials selected from the group consisting of salts, polymers and mixtures thereof. Useful aggregate inhibiting materials include, divalent salts such as magnesium salts, for example, magnesium chloride, magnesium acetate, magnesium phosphate, magnesium formate, magnesium boride, magnesium titanate, magnesium sulfate heptahydrate; calcium salts, for example, calcium chloride, calcium formate, calcium calcium acetate, calcium bromide; trivalent salts, such as aluminum salts, for example, aluminum sulfate, aluminum phosphate, aluminum chloride n-hydrate and polymers that have the ability to suspend anionic particles such as soil suspension polymers, for example, (polyethylene imines, alkoxylated polyethylene imines, polyquaternium-6 and polyquaternium-7.

In one aspect of the invention, encapsulated benefit agents are manufactured and are subsequently coated with a material to reduce the rate of leakage of the benefit agent from the particles when the particles are subjected to a bulk environment containing, for example, surfactants, polymers, and solvents. Non-limiting examples of coating materials that can serve as barrier materials include materials selected from the group consisting of polyvinyl pyrrolidone homopolymer, and its various copolymers with styrene, vinyl acetate, imidazole, primary and secondary amine containing monomers, methyl acrylate, polyvinyl acetal, maleic anhydride; polyvinyl alcohol homopolymer, and its various copolymers with vinyl acetate, 2-acrylamide-2-methylpropane sulfonate, primary and secondary amine containing monomers, imidazoles, methyl acrylate; polyacrylamides; polyacrylic acids; microcrystalline waxes; paraffin waxes; modified polysaccharides such as waxy maize or dent corn starch, octenyl succinated starches, derivatized starches such as hydroxyethylated or hydroxypropylated starches, carrageenan, guar gum, pectin, xanthan gum; modified celluloses such as hydrolyzed cellulose acetate, hydroxy propyl cellulose, methyl cellulose, and the like; modified proteins such as gelatin; hydrogenated and non-hydrogenated polyalkenes; fatty acids; hardened shells such as urea crosslinked with formaldehyde, gelatin-polyphosphate, melamine-formaldehyde, polyvinyl alcohol crosslinked with sodium tetraborate or gluteraldehyde; latexes of styrene-butadiene, ethyl cellulose, inorganic materials such as clays including magnesium silicates, aluminosilicates; sodium silicates, and the like; and mixtures thereof. Such materials can be obtained from CP Kelco Corp. of San Diego, California, USA; Degussa AG or Dusseldorf, Germany; BASF AG of Ludwigshafen, Germany; Rhodia Corp. of Cranbury, New Jersey, USA; Baker Hughes Corp. of Houston, Texas, USA; Hercules Corp. of Wilmington, Delaware, USA; Agrium Inc. of Calgary, Alberta, Canada, ISP of New Jeresy U.S.A..

Suitable equipment for use in the processes disclosed herein may include continuous stirred tank reactors, homogenizers, turbine agitators, recirculating pumps, paddle mixers, ploughshear mixers, ribbon blenders, vertical axis granulators and drum mixers, both in batch and, where available, in continuous process configurations, spray dryers, and extruders. Such equipment can be obtained from Lodige GmbH (Paderborn, Germany), Littleford Day, Inc. (Florence, Kentucky, U.S.A.), Forberg AS (Larvik, Norway), Glatt Ingenieurtechnik GmbH (Weimar, Germany), Niro (Soeborg, Denmark), Hosokawa Bepex Corp. (Minneapolis, Minnesota, USA), Arde Barinco (New Jersey, USA).

Formaldehyde Scavenging

In one aspect, encapsulated benefit agent may be combined with a formaldehyde scavenger. In one aspect, encapsulated benefit agent may comprise the encapsulated benefit agent of the present invention. Suitable formaldehyde scavengers include materials selected from the group consisting of sodium bisulfite, urea, ethylene urea, cysteine, cysteamine, lysine, glycine, serine, carnosine, histidine, glutathione, 3,4-diaminobenzoic acid, allantoin, glycouril, anthranilic acid, methyl anthranilate, methyl 4-aminobenzoate, ethyl acetoacetate, acetoacetamide, malonamide, ascorbic acid, 1,3-dihydroxyacetone dimer, biuret, oxamide, benzoguanamine, pyroglutamic acid, pyrogallol, methyl gallate, ethyl gallate, propyl gallate, triethanol amine, succinamide, thiabendazole, benzotriazol, triazole, indoline, sulfanilic acid, oxamide, sorbitol, glucose, cellulose, poly(vinyl alcohol), partially hydrolyzed poly(vinylformamide), poly(vinyl amine), poly(ethylene imine), poly(oxyalkyleneamine), poly(vinyl alcohol)-co-poly(vinyl amine), poly(4-aminostyrene), poly(1-lysine), chitosan, hexane diol, ethylenediamine-N,N'-bisacetoacetamide, N-(2-ethylhexyl)acetoacetamide, 2-benzoylacetoacetamide, N-(3-phenylpropyl)acetoacetamide, lilial, helional, melonal, triplal, 5,5-dimethyl-1,3-cyclohexanedione, 2,4-dimethyl-3-cyclohexenecarboxaldehyde, 2,2-dimethyl-1,3-dioxan-4,6-dione, 2-pentanone, dibutyl amine, triethylenetetramine, ammonium hydroxide, benzylamine, hydroxycitronellol, cyclohexanone, 2-butanone, pentane dione, dehydroacetic acid, or a mixture thereof. These formaldehyde scavengers may be obtained from Sigma/Aldrich/Fluka of St. Louis, MO. U.S.A. or PolySciences, Inc. of Warrington, PA U.S.A.

Such formaldehyde scavengers are typically combined with a slurry containing said benefit agent containing delivery particle, at a level, based on total slurry weight, of from about 2 wt.% to about 18 wt.%, from about 3.5 wt.% to about 14 wt.% or even from about 5 wt.% to about 13 wt.%.

In one aspect, such formaldehyde scavengers may be combined with a product containing a benefit agent containing delivery particle, said scavengers being combined with said product at a level, based on total product weight, of from about 0.005% to about 0.8%, alternatively from about 0.03% to about 0.5%, alternatively from about 0.065% to about 0.25% of the product formulation,.

In another aspect, such formaldehyde scavengers may be combined with a slurry containing said encapsulated benefit agent, at a level, based on total slurry weight, of from about 2 wt.% to about 14 wt.%, from about 3.5 wt.% to about 14 wt.% or even from about 5 wt.% to about 14 wt.% and said slurry may be added to a product matrix to which addition an identical or different scavenger may be added at a level, based on total product weight, of from about 0.005% to about 0.5%, alternatively from about 0.01% to about 0.25%, alternatively from about 0.05% to about 0.15% of the product formulation,

In one aspect, one or more of the aforementioned formaldehyde scavengers may be combined with a consumer product containing an encapsulated benefit agent at a level, based on total liquid fabric enhancing product weight, of from 0.005% to about 0.8%, alternatively from about 0.03% to about 0.4%, alternatively from about 0.06% to about 0.25% of the product formulation

In one aspect, such formaldehyde scavengers may be combined with a liquid laundry detergent product containing a benefit agent containing delivery particle, said scavengers being selected from the group consisting of sodium bisulfite, urea, ethylene urea, cysteine, cysteamine, lysine, glycine, serine, carnosine, histidine, glutathione, 3,4-diaminobenzoic acid, allantoin, glycouril, anthranilic acid, methyl anthranilate, methyl 4-aminobenzoate, ethyl acetoacetate, acetoacetamide, malonamide, ascorbic acid, 1,3-dihydroxyacetone dimer, biuret, oxamide, benzoguanamine, pyroglutamic acid, pyrogallol, methyl gallate, ethyl gallate, propyl gallate, triethanol amine, succinamide, thiabendazole, benzotriazol, triazole, indoline, sulfanilic acid, oxamide, sorbitol, glucose, cellulose, poly(vinyl alcohol), partially hydrolyzed poly(vinylformamide), poly(vinyl amine), poly(ethylene imine), poly(oxyalkyleneamine), poly(vinyl alcohol)-co-poly(vinyl amine), poly(4-aminostyrene), poly(1-lysine), chitosan, hexane diol, ethylenediamine-N,N'-bisacetoacetamide, N-(2-ethylhexyl)acetoacetamide, 2-benzoylacetoacetamide, N-(3-phenylpropyl)acetoacetamide, lilial, helional, melonal, triplal, 5,5-dimethyl-1,3-cyclohexanedione, 2,4-dimethyl-3-cyclohexenecarboxaldehyde, 2,2-dimethyl-1,3-dioxan-4,6-dione, 2-pentanone, dibutyl amine, triethylenetetramine, ammonium hydroxide, benzylamine, hydroxycitronellol, cyclohexanone, 2-butanone, pentane dione, dehydroacetic acid and mixtures thereof, and combined with said liquid laundry detergent product at a level, based on total liquid laundry detergent product weight, of from about 0.003 wt.% to about 0.20 wt.%, from about 0.03 wt.% to about 0.20 wt.% or even from about 0.06 wt.% to about 0.14 wt.%.

In one aspect, such formaldehyde scavengers may be combined with a hair conditioning product containing a benefit agent containing delivery particle, at a level, based on total hair conditioning product weight, of from about 0.003 wt. % to about 0.30 wt.%, from about 0.03 wt.% to about 0.20 wt.% or even from about 0.06 wt.% to about 0.14 wt.%., said selection of scavengers being identical to the list of scavengers in the previous paragraph relating to a liquid laundry detergent product.

Compositions Comprising Benefit Agent Containing Delivery Particles

Applicants' consumer products may comprise an embodiment of the benefit agent delivery composition disclosed in the present application. In one aspect, said consumer products may be a powdered, granule or other essentially dry detergent.

In one aspect, a consumer product that may comprise one or more of the benefit agent delivery compositions of the present invention and an adjunct ingredient is disclosed.

In one aspect of the aforementioned consumer product, said consumer product adjunct may be selected from the group consisting of polymers, for example cationic polymers, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments and mixtures thereof.

In one aspect of the aforementioned consumer product, said consumer product may comprise a total of, based on total consumer product weight, from about 0.1% to about 20%, from about 0.2% to about 15%, from about 0.3% to about 10%, from about 0.4% to about 8%, or even from about 0.5% to about 5% of one or more of the benefit agent delivery compositions of the present invention.

In one aspect of the aforementioned consumer product, said consumer product may comprise one or more of the benefit agent delivery compositions of the present invention and a material selected from the group consisting of dyes; perfume; optical brighteners; deposition aids; and mixtures thereof.

Aspects of the invention include the use of the benefit agent delivery composition of the present invention in laundry detergent compositions (e.g., TIDE™), hard surface cleaners (e.g., MR CLEAN™), automatic dishwashing liquids (e.g., CASCADE™), and floor cleaners (e.g., SWIFFER™). Non-limiting examples of cleaning compositions may include those described in U.S. Pat. Nos. 4,515,705; 4,537,706; 4,537,707; 4,550,862; 4,561,998; 4,597,898; 4,968,451; 5,565,145; 5,929,022; 6,294,514; and 6,376,445. The cleaning compositions disclosed herein are typically formulated such that, during use in aqueous cleaning operations, the wash water will have a pH of between about 6.5 and about 12, or between about 7.5 and 10.5. Liquid dishwashing product formulations typically have a pH between about 6.8 and about 9.0. Cleaning products are typically formulated to have a pH of from about 7 to about 12. Techniques for controlling pH at recommended usage levels include the use of buffers, alkalis, acids, etc., and are well known to those skilled in the art.

Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance performance, for treatment of the substrate to be cleaned, or to modify the aesthetics of the composition as is the case with perfumes, colorants, dyes or the like. It is understood that such adjuncts are in addition to the components that are supplied via Applicants' agglomerate/particle. The precise nature of these additional components, and levels of incorporation thereof, will depend on the physical form of the composition and the nature of the operation for which it is to be used. Suitable adjunct materials include, but are not limited to, polymers, for example cationic polymers, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812 B1 and 6,326,348 B1 that are incorporated by reference.

As stated, the adjunct ingredients are not essential to Applicants' cleaning and fabric care compositions. Thus, certain embodiments of Applicants' compositions do not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents, fabric softeners, carriers, hydrotropes, processing aids and/or pigments. However, when one or more adjuncts are present, such one or more adjuncts may be present as detailed below:

Surfactants - The compositions according to the present invention can comprise a surfactant or surfactant system wherein the surfactant can be selected from nonionic and/or anionic and/or cationic surfactants and/or ampholytic and/or zwitterionic and/or semi-polar nonionic surfactants. The surfactant is typically present at a level of from about 0.1%, from about 1%, or even from about 5% by weight of the cleaning compositions to about 99.9%, to about 80%, to about 35%, or even to about 30% by weight of the cleaning compositions.

Builders - The compositions of the present invention can comprise one or more detergent builders or builder systems. When present, the compositions will typically comprise at least about 1% builder, or from about 5% or 10% to about 80%, 50%, or even 30% by weight, of said builder. Builders include, but are not limited to, the alkali metal, ammonium and alkanolammonium salts of polyphosphates, alkali metal silicates, alkaline earth and alkali metal carbonates, aluminosificate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Chelating Agents - The compositions herein may also optionally contain one or more copper, iron and/or manganese chelating agents. If utilized, chelating agents will generally comprise from about 0.1% by weight of the compositions herein to about 15%, or even from about 3.0% to about 15% by weight of the compositions herein.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in the compositions herein, the dye transfer inhibiting agents are present at levels from about 0.0001%, from about 0.01%, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The cleaning compositions can comprise one or more enzymes which provide cleaning performance and/or fabric care benefits. Examples of suitable enzymes include, but are not limited to, hemicellulases, cellulases, cellobiose dehydrogenases, peroxidases, proteases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, and amylases, or mixtures thereof. A typical combination is an enzyme cocktail that may comprise, for example, a protease and lipase in conjunction with amylase. When present in a cleaning composition, the aforementioned additional enzymes may be present at levels from about 0.00001% to about 2%, from about 0.0001% to about 1% or even from about 0.001% to about 0.5% enzyme protein by weight of the composition.

Suitable enzymes are commercially available from Genencor International Inc., Palo Alto, California, AB Enzymes GmbH, Darmstadt, Germany and Novozymes A/S, Bagsvaerd, Denmark and include Alcalase®, Savinase®, Primase®, Durazym®, Polarzyme®, Kannase®, Liquanase®, Ovozyme®, Neutrase®, Everlase®, Esperase®, Maxatase®, Maxacal®, Maxapem®, Properase®, Purafect®, Purafect Prime®, Purafect Ox®, FN3®, FN4®, Excellase® and Purafect OXP®, DURAMYL®, LIQUEZYME® TERMAMYL®, TERMAMYL ULTRA®, NATALASE®, SUPRAMYL®, STAINZYME®, STAINZYME PLUS®, STAINZYME ULTRA®, FUNGAMYL®, BAN® Lipolase®, Lipolase Ultra®, Lipoprime®, Lipex®, Mannaway®, Pectaway®, Pectawash®, Purabrite®, Celluclean®, Carezyme®, Celluzyme®, Biotouch®, Endolase® and Puradax HA®.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra (methyl-enephosphonic acid) and water-soluble salts thereof. Such catalysts are disclosed in U.S. patent 4,430,243.

If desired, the compositions herein can be catalyzed by means of a manganese compound. Such compounds and levels of use are well known in the art and include, for example, the manganese-based catalysts disclosed in U.S. patent 5,576,282.

Cobalt bleach catalysts useful herein are known, and are described, for example, in U.S. patents 5,597,936 and 5,595,967. Such cobalt catalysts are readily prepared by known procedures, such as taught for example in U.S. patents 5,597,936, and 5,595,967.

Compositions herein may also suitably include a transition metal complex of a macropolycyclic rigid ligand - abreviated as "MRL". As a practical matter, and not by way of limitation, the compositions and cleaning processes herein can be adjusted to provide on the order of at least one part per hundred million of the benefit agent MRL species in the aqueous washing medium, and may provide from about 0.005 ppm to about 25 ppm, from about 0.05 ppm to about 10 ppm, or even from about 0.1 ppm to about 5 ppm, of the MRL in the wash liquor.

Preferred transition-metals in the instant transition-metal bleach catalyst include manganese, iron and chromium. Preferred MRL's herein are a special type of ultra-rigid ligand that is cross-bridged such as 5,12-diethyl-1,5,8,12-tetraazabicyclo[6.6.2]hexa-decane.

Suitable transition metal MRLs are readily prepared by known procedures, such as taught for example in WO 00/32601, and U.S. patent 6,225,464.

Processes of Making and Using Compositions

The compositions of the present invention can be formulated into any suitable form and prepared by any process chosen by the formulator, non-limiting examples of which are described in U.S. 5,879,584; U.S. 5,691,297; U.S. 5,574,005; U.S. 5,569,645; U.S. 5,565,422; U.S. 5,516,448; U.S. 5,489,392; U.S. 5,486,303 all of which are incorporated herein by reference.

Method of Use

Compositions containing the encapsulated benefit agent disclosed herein can be used to clean or treat a situs *inter alia* a surface or fabric. Typically at least a portion of the situs is contacted with an embodiment of Applicants' composition, in neat form or diluted in a liquor, for example, a wash liquor and then the situs may be optionally washed and/or rinsed. In one aspect, a situs is optionally washed and/or rinsed, contacted with a one or more of the benefit agent delivery compositions of the present invention or a consumer product comprising one or more of the benefit agent delivery compositions of the present invention and then optionally washed and/or rinsed. For purposes of the present invention, washing includes but is not limited to, scrubbing, and mechanical agitation. The fabric may comprise most any fabric capable of being laundered or treated in normal consumer use conditions. Liquors that may comprise the disclosed compositions may have a pH of from about 3 to about 11.5. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1.

TEST METHODS

It is understood that the test methods that are disclosed in the Test Methods Section of the present application should be used to determine the respective values of the parameters of Applicants' invention as such invention is described and claimed herein.

Method to Determine the Characteristic Dimension (Agglomerate and Particulates)

The particle size distribution of the benefit agent delivery composition is determined using the ASTM test method E726-01 "Particle Size Distribution of Granular Carriers and Granular Pesticides". Specifically the method should be carried out using a Tyler RoTap sieve shaker supplied with cast iron sieve stack lid with centrally mounted cork (W.S. Tyler Company, Cleveland, Ohio). At least 5 sieve sizes should be selected to cover the full particle size range of the material being analysed (step 7.1). If after sieving, more than 40wt% of the sample is found on a single sieve then the sieve selection should be modified and the sample retested until <40wt% is found on all sieves.

The following parameters for operation of the RoTap should be applied to step 7.4 of the method:

1) 152 taps/minute
2) 285 rpm elliptical motion
3) Cork on lid to protrude 5 mm from top of holding cup.
4) A hammer drop of 33 mm from the peak height of the hammer to the top of the cork.
5) Sieve time 5 minutes.

The data are plotted on a semi-log plot with the micron size opening of each sieve plotted against the logarithmic abscissa and the cumulative mass percent (Q3) plotted against the linear ordinate. An example of the above data representation is given in ISO 9276-1:1998, "Representation of results of particle size analysis - Part 1: Graphical Representation", Figure A.4. The seed material median particle size (D50), for the purpose of this invention, is defined as the abscissa value at the point where the cumulative mass percent is equal to 50 percent, and is calculated by a straight line interpolation between the data points directly above (a50) and below (b50) the 50% value using the following equation:

$$D50 = 10\text{^}[\text{Log}(Da50) - (\text{Log}(Da50) - \text{Log}(Db50))*(Qa50 - 50\%)/(Qa50 - Qb50)]$$

where Qa50 and Qb50 are the cumulative mass percentile values of the data immediately above and below the 50th percentile, respectively; and Da50 and Db50 are the micron sieve size values corresponding to these data. The median particle size on a mass basis is considered, for purposes of the present application, to be the characteristic dimension.

Method to Determine the Characteristic Dimension (Extrudates)

The diameter of an extrudate is obtained by measurement using a micrometer while the material is at 20°C. In order to determine the mean, five representative extrudates are taken from the sample to be tested and are measured taking care to not deform the extrudates during the measuring process and the arithmetic mean of such measurements is then calculated. Such arithmetic mean is considered, for purposes of the present application, to be the characteristic dimension of the extrudates.

EXAMPLES

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

EXAMPLE 1: 85% Core / 15wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.
178 grams of the capsule core material which comprise a fragrance oil is added to the first mixture at a temperature of 45°C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 16 micron. The temperature of the mixture is gradually raised to 65 degrees Centigrade, and is maintained at this temperature overnight with continuous stirring to initiate and complete encapsulation.
To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 2: 90% Core / 10wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.
280 grams of the capsule core material which comprise a fragrance oil is added to the first mixture at a temperature of 45°C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 18 micron. The temperature of the mixture is gradually raised to 65 degrees Centigrade, and is maintained at this temperature overnight with continuous stirring to initiate and complete encapsulation.
To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 3: 80% Core / 20wt% Wall Melamine based Polyurea capsule

A first mixture is prepared by combining 208 grams of water and 5 grams of alkyl acrylate-acrylic acid copolymer (Polysciences, Inc. of Warrington, Pennsylvania, USA). This first mixture is adjusted to pH 5.0 using acetic acid.

125 grams of the capsule core material which comprises a fragrance oil is added to the first mixture at a temperature of 45°C to form an emulsion. The ingredients to form the capsule wall material are prepared as follows: 9 grams of a corresponding capsule wall material copolymer pre-polymer (butylacrylate-acrylic acid copolymer) and 90 grams of water are combined and adjusted to pH 5.0. To this mixture is added 28 grams of a partially methylated methylol melamine resin solution ("Cymel 385", 80% solids, Cytec). This mixture is added to the above described fragrance oil-in-water emulsion with stirring at a temperature of 45 degrees Centigrade. High speed blending is used to achieve a volume-mean particle size of 15 micron. The temperature of the mixture is gradually raised to 65 degrees Centigrade, and is maintained at this temperature overnight with continuous stirring to initiate and complete encapsulation.

To form the acrylic acid-alkyl acrylate copolymer capsule wall, the alkyl group can be selected from ethyl, propyl, butyl, amyl, hexyl, cyclohexyl, 2-ethylhexyl, or other alkyl groups having from one to about sixteen carbons, preferably one to eight carbons.

EXAMPLE 4

9kg aliquots of perfume microcapsule slurry of Examples 1 to 3 are mixed using a Eurostar mixer (IKA) with a R1382 attachment at a constant speed of 200 RPM. To each of the perfume microcapsule slurries, 500g of carboxymethyl cellulose (CP Kelco) is added while mixing. These slurries are mixed for a total of two hours or until a uniform paste is formed. Thus three separate pastes are produced.

EXAMPLE 5

1.28kg of precipitated silica Sipernat® 22S (Degussa) is added to a F-20 paddle mixer (Forberg). The mixer is run initially for 5 seconds to distribute the silica evenly on the base of the mixer. The mixer is stopped and 8.25kg of paste, made according to Example 4, is evenly distributed onto the powder. The mixer is then run at 120rpm for a total of 30 seconds. Following mixing, the wet particles are dumped out of the mixer and screened using a 2000 micron sieve to remove the oversize. The good product passing through the screen is dried in 500g batches in a CDT 0.02 fluid bed dryer (Niro) to a final moisture content of 20wt% measured by Karl Fischer. The dryer is operated at an inlet temperature of 140°C and air velocity of 0.68m/s. The procedure is repeated for each of the pastes produced in Example 4 to yield three separate agglomerates.

EXAMPLE 6

800g aliquots of perfume microcapsule slurry, of Examples 1 to 3, are each mixed together with 200g of carboxymethyl cellulose (CP Kelco) using a spatula until a uniform paste has been formed. Thus three separate pastes are produced. Each paste is then passed through an APV 19mm barrel twin screw extruder (Baker Perkins Ltd.) using conveying screws only and a die plate with 1mm diameter holes. The extruder is run at a speed of 100rpm and at ambient temperature to form three sets of extrudates.
Following the extrusion operation, each set of extrudates are spread out as a thin layer onto separate trays and placed in an oven to dry at 50°C until they reach a moisture content of 20wt%. Each set of dried extrudates are then broken up by hand and sieved through a 2000 micron screen to remove the majority of the longer extrudates.
The efficiency of the process may be improved via the addition of a dusting agent following the extrusion step to avoid coagulation during drying.

EXAMPLE 7

1kg aliquots of perfume microcapsule slurry, of Examples 1 to 3, are each mixed with 100g of a cross-linked polyacrylate (Aqualic CA-Series, Nippon Shokubai) using a spatula until the mix forms a wet mass of discrete particles. Each set of wet particles is placed into separate plastic bags and dusted with 300g of Zeolite 4A (Industrial Chemicals Ltd), until free flowing agglomerate particles are formed. Following mixing, the material is screened using a 2000 micron sieve to remove the oversize.

EXAMPLE 8

250g aliquots of perfume microcapsule slurry, of Examples 1 to 3, are each mixed with 5g of a cross-linked polyacrylate (Aqualic CA-Series, Nippon Shokubai) using a spatula until the mix forms a paste. 100g of each paste is separately mixed together with 50g of Zeolite 4A (Industrial Chemicals Ltd) in a kitchen food processor (Braun) thus forming three sets of agglomerate particles. Following mixing, each set of agglomerate particles is screened using a 2000 micron sieve to remove the oversize. The good product passing through the screen for each agglomerate particle set is dried by spreading a thin layer of the material on a tray and placing in an oven at 50°C until the agglomerate particles reach a moisture content of 20wt%.

EXAMPLE 9

Sodium alginate powder (Manucol DM, International Speciality Products) is dissolved in three separate aliquots of perfume microcapsule slurry from Examples 1 to 3 to form 1% alginate solutions. These compositions are mixed thoroughly using an Ultra Turrax T25 mixer (IKA) at a speed of 10000/min. The PMC/alginate mixtures are then added drop wise to separate 1% aqueous solutions of chitosan (Primex). The beads remain in the chitosan solution until they have cured, typically 10 - 45 minutes (cure time depends on grade and concentration of chitosan used). Following the curing step, the material is removed and dried at 37°C to a moisture content of 12wt% thus producing three separate sets of beads.

EXAMPLE 10

Sodium alginate powder (Manucol DM, International Speciality Products) is dissolved in three separate aliquots of perfume microcapsule slurry from Examples 1 to 3 to form 1% alginate solutions. These compositions are mixed thoroughly using an Ultra Turrax T25 mixer (IKA) at a speed of 10000/min. The PMC/alginate mixtures are then added drop wise to separate 1% aqueous solution of calcium chloride (Sigma-Aldrich). After 5 minutes, the material is removed and dried at 37°C to a moisture content of 12wt% thus producing three separate sets of beads.

EXAMPLE 11

110g of Lupasol® WF (BASF) is added to a beaker and heated to about 60°C using a water bath. Upon reaching this temperature, 150g of delta damascone (IFF) is added to the Lupasol® WF (BASF) and mixed using an IKA Ultra Turrax T25 mixer (IKA) at a speed of 20000/min until homogeneous reaction product (typically known as an amine reaction product) is formed. During this time the beaker is kept in the water bath at approximately 60°C. Following mixing the material is maintained at this temperature for a further 12 hours. After which time, 250g of perfume microcapsule slurry from Example 1, pre-heated to about 60°C, is added together with 425g of molten Lutensol® AT 80 powder (BASF) melted at around 70°C. This mixture is blended using an IKA Ultra Turrax T25 mixer (IKA) at a speed of 20000/min at approximately 60°C until homogeneous. 100g of the blended mixture is added to 167g of light sodium carbonate and mixed in a kitchen food processor (Braun) to form agglomerates.

The procedure is repeated for each of the perfume microcapsule slurries from Examples 1 to 3 to create three different agglomerate particles.

EXAMPLES 12-19

Examples of laundry detergent compositions comprising the perfume composition are included below.

| Raw material | %w/w of laundry detergent compositions | | | | | | | |
|---|---|---|---|---|---|---|---|---|
| | 12 | 13 | 14 | 15 | 16 | 17 | 18 | 19 |
| Linear alkyl benzene sulphonate | 7.1 | 6.7 | 11.0 | 10.6 | 6.9 | 4.5 | 10.1 | 8.9 |
| Sodium $C_{12-15}$ alkyl ethoxy sulphate having a molar average degree of ethoxylation of 3 | 3.5 | 0.0 | 1.5 | 0.0 | 0.0 | 0.0 | 0.0 | 1.9 |
| Acrylic Acid/Maleic Acid Copolymer | 3.6 | 1.8 | 4.9 | 2.0 | 1.0 | 1.6 | 3.9 | 2.3 |
| Sodium Alumino Silicate (Zeolite 4A) | 4.0 | 0.5 | 0.8 | 1.4 | 16.3 | 0.0 | 17.9 | 2.4 |
| Sodium Tripolyphosphate | 0.0 | 17.5 | 0.0 | 15.8 | 0.0 | 23.3 | 0.0 | 0.0 |
| Sodium Carbonate | 23.2 | 16.8 | 30.2 | 17.3 | 18.4 | 9.0 | 20.8 | 30.0 |
| Sodium Sulphate | 31.4 | 29.4 | 35.5 | 7.2 | 26.3 | 42.8 | 33.2 | 28.3 |
| Sodium Silicate | 0.0 | 4.4 | 0.0 | 4.5 | 0.0 | 6.1 | 0.0 | 4.6 |
| $C_{14-15}$ alkyl ethoxylated alcohol having a molar average degree of ethoxylation of 7 | 0.4 | 2.6 | 0.8 | 2.5 | 3.1 | 0.3 | 3.8 | 0.4 |

(continued)

| Raw material | %w/w of laundry detergent compositions | | | | | | | |
|---|---|---|---|---|---|---|---|---|
| | 12 | 13 | 14 | 15 | 16 | 17 | 18 | 19 |
| Sodium Percarbonate | 16.0 | 0.0 | 8.4 | 20.4 | 13.1 | 3.6 | 0.0 | 7.0 |
| Sodium Perborate | 0.0 | 9.9 | 0.0 | 0.0 | 0.0 | 0.0 | 0.0 | 0.0 |
| Tetraacetylethylenediamine (TAED) | 2.2 | 1.7 | 0.0 | 4.7 | 3.6 | 0.0 | 0.0 | 0.8 |
| Calcium Bentonite | 0.0 | 0.0 | 0.0 | 1.8 | 0.0 | 0.0 | 0.0 | 5.6 |
| Citric acid | 2.0 | 1.5 | 2.0 | 2.0 | 2.5 | 1.0 | 2.5 | 1.0 |
| Protease (84mg active/g) | 0.14 | 0.12 | 0.0 | 0.12 | 0.09 | 0.08 | 0.10 | 0.08 |
| Amylase (22mg active/g) | 0.10 | 0.11 | 0.0 | 0.10 | 0.10 | 0.0 | 0.14 | 0.08 |
| Lipase (11mg active/g) | 0.70 | 0.50 | 0.0 | 0.70 | 0.50 | 0.0 | 0.0 | 0.0 |
| Cellulase (2.3mg active/g) | 0.0 | 0.0 | 0.0 | 0.0 | 0.0 | 0.0 | 0.18 | 0.0 |
| Benefit agent composition of Example 5 | 1.4 | 0.6 | 0.8 | 1.0 | 0.7 | 0.3 | 0.7 | 1.2 |
| Water & Miscellaneous | Balance to 100% | | | | | | | |

The equipment and materials described in Examples 1 through to 19 can be obtained from the following: IKA Werke GmbH & Co. KG, Staufen, Germany; CP Kelco, Atlanta, United States; Forberg International AS, Larvik, Norway; Degussa GmbH, Düsseldorf, Germany; Niro A/S, Soeberg, Denmark; Baker Perkins Ltd, Peterborough, United Kingdom; Nippon Shokubai, Tokyo, Japan; BASF, Ludwigshafen, Germany; Braun, Kronberg, Germany; Industrial Chemicals Limited, Thurrock, United Kingdom; Primex ehf, Siglufjordur, Iceland; ISP World Headquarters; Polysciences, Inc. of Warrington, Pennsylvania, United States; Cytec Industries Inc., New Jersey, United States; International Specialty Products, Wayne, New Jersey, United States; P&G Chemicals Americas, Cincinnati, Ohio, United States; Sigma-Aldrich Corp., St. Louis, Missouri, United States.

The dimensions and values disclosed herein are not to be understood as being strictly limited to the exact numerical values recited. Instead, unless otherwise specified, each such dimension is intended to mean both the recited value and functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

## Claims

1. A process of producing a cleaning composition comprising a benefit agent delivery composition in the form of an agglomerate, particulate or extrudate and an adjunct ingredient, said process comprising:

a) combining (i) an encapsulated benefit agent, wherein said encapsulated benefit agent comprises a perfume microcapsule, said perfume microcapsule comprising a shell, said shell comprising cross-linked melamine formaldehyde (ii) a plasticizer, wherein said plasticizer comprises water and iii) a binder, wherein said binder is selected from the group consisting of celluloses preferably methylcellulose, more preferably CMC, and derivatives thereof; alginate and derivatives thereof; starches; polyvinyl alcohols; polyethylene oxide; polyvinylpyrolidone; polysaccharides preferably chitosan and/or natural gums preferably carrageenan; polyacrylates preferably cross-linked polyacrylates; waxes; polyethylene glycols having a weight average molecular weight of greater than 4000 Da or even from 4000 Da to 15,000 Da; alcohol ethoxylates; surfactants and mixtures thereof to form a mixture;
b) combining said mixture with a dusting agent, wherein said dusting agent comprises a material selected from the group consisting of silicas; aluminosilicates preferably zeolite; clays; and mixtures thereof to form a material; and

c) removing a sufficient amount of said plasticizer from said material to yield a benefit agent delivery composition in the form of an agglomerate, particulate or extrudate comprising, based on total weight, delivery composition from 2% to 97% by weight encapsulated benefit agent comprising a sufficient amount of benefit agent to provide, based on total weight of benefit delivery composition, from 1 % to 85% benefit agent, from 1 % to 50% by weight, preferably from 2% to 45% by weight plasticizer, from 1 to 50 wt% binder and from 5%-50% by weight dusting agent; and

d) mixing with an adjunct ingredient to form a cleaning composition.

2. The process according to any preceding claim said agglomerate, extrudate or particulate having a characteristic dimension of 100 microns to 3000 microns, more preferably from 200 microns to 2500 microns, more preferably from 300 microns to 2000 microns, more preferably from 400 microns to 1400 microns, most preferably from 500 microns to 1200 microns, wherein for said agglomerates and particulates said characteristic dimension is the median particle size of said agglomerates and particulates and the characteristic dimension of said extrudates is the mean diameter of said extrudates.

3. A process according to any preceding claim for making a cleaning composition comprising a benefit agent delivery composition of any preceding claim in an amount based on total cleaning composition product weight, from 0.1 % to 20%, and an adjunct ingredient, preferably selected from the group consisting of polymers, (for example cationic polymers), surfactants, builders, chelating agents, dye transfer inhibiting agents, dispersants, enzymes, and enzyme stabilizers, catalytic materials, bleach activators, polymeric dispersing agents, clay soil removal/antiredeposition agents, brighteners, suds suppressors, dyes, additional perfume and perfume delivery systems, structure elasticizing agents, fabric softeners, carriers, hydrotropes, processing aids and/or pigments and mixtures thereof.

4. A process according to claim 3 in which the adjunct ingredient comprises a material selected from the group consisting of dyes; perfume; optical brighteners; deposition aids; and mixtures thereof.

**Patentansprüche**

1. Verfahren zum Herstellen einer Reinigungszusammensetzung, umfassend eine Wirkstoffabgabezusammensetzung in Agglomerat-, Partikel- oder Extrudatform und einen Zusatzstoff, wobei das Verfahren Folgendes umfasst:

a) Kombinieren (i) eines verkapselten Wirkstoffs, wobei der verkapselte Wirkstoff eine Parfümmikrokapsel umfasst, wobei die Parfümmikrokapsel einen Mantel umfasst, wobei der Mantel quervernetztes Melaminformaldehyd umfasst, (ii) eines Weichmachers, wobei der Weichmacher Wasser umfasst, und (iii) eines Bindemittels, wobei das Bindemittel ausgewählt wird aus der Gruppe bestehend aus Cellulosen, vorzugsweise Methylcellulose, mehr bevorzugt CMC, und Derivaten davon; Alginat und Derivaten davon; Stärken; Polyvinylalkoholen; Polyethylenoxid; Polyvinylpyrolidon; Polysacchariden, vorzugsweise Chitosan und/oder Naturgummistoffen, vorzugsweise Carrageen; Polyacrylaten, vorzugsweise quervernetzten Polyacrylaten; Wachsen; Polyethylenglycolen mit einem gewichtsmittleren Molekulargewicht von mehr als 4000 Da oder sogar von 4000 Da bis 15.000 Da; Alkoholethoxylaten; Tensiden und Gemischen davon, um ein Gemisch zu bilden;

b) Kombinieren des Gemisches mit einem Stäubemittel, wobei das Stäubemittel ein Material umfasst, das ausgewählt wird aus der Gruppe bestehend aus Silicas; Aluminosilicaten, vorzugsweise Zeolith; Tonen; und Gemischen davon, um ein Material zu bilden; und

c) Entfernen einer ausreichenden Menge des Weichmachers aus dem Material, um eine Wirkstoffabgabezusammensetzung in Agglomerat-, Partikel- oder Extrudatform zu ergeben, die, basierend auf dem Gesamtgewicht, die Abgabezusammensetzung von 2 bis 97 Gew.-% des verkapselten Wirkstoffs umfasst, umfassend eine ausreichende Menge des Wirkstoffs, um basierend auf dem Gesamtgewicht der Wirkstoffabgabezusammensetzung, von 1 bis 85 Gew.-% Wirkstoff, von 1 bis 50 Gew.-%, vorzugsweise von 2 bis 45 Gew.-% Weichmacher, von 1 bis 50 Gew.-% Bindemittel und von 5 bis 50 Gew-.% Stäubemittel bereitzustellen; und

d) Vermischen mit einem Zusatzbestandteil, um eine Reinigungszusammensetzung zu bilden.

2. Verfahren nach einem der vorstehenden Ansprüche, wobei das Agglomerat, das Extrudat oder die Partikel eine charakteristische Abmessung von 100 Mikrometern bis 3000 Mikrometern, mehr bevorzugt von 200 Mikrometern bis 2500 Mikrometern, mehr bevorzugt von 300 Mikrometern bis 2000 Mikrometern, mehr bevorzugt von 400 Mikrometern bis 1400 Mikrometern, am meisten bevorzugt von 500 Mikrometern bis 1200 Mikrometern aufweisen, wobei für die Agglomerate und Partikel die charakteristische Abmessung die mittlere Partikelgröße der Agglomerate und Partikel ist und die charakteristische Abmessung der Extrudate der mittlere Durchmesser der Extrudate ist.

**3.** Verfahren nach einem der vorstehenden Ansprüche zum Herstellen einer Reinigungszusammensetzung, die eine Wirkstoffabgabezusammensetzung nach einem der vorstehenden Ansprüche in einer Menge basierend auf dem Gesamtproduktgewicht der Reinigungszusammensetzung von 0,1 bis 20 % und einen Zusatzstoff umfasst, vorzugsweise ausgewählt aus der Gruppe bestehend aus Polymeren (beispielsweise kationischen Polymeren), Tensiden, Gerüststoffen, Chelatbildnern, Anti-Farbstoffübertragungsmitteln, Dispersionsmitteln, Enzymen und Enzymstabilisatoren, katalytischen Materialien, Bleichaktivierungsmitteln, Polymerdispersionsmitteln, Tonerdeentfernungs-/Anti-Wiederablagerungsmitteln, Aufhellern, Schaumunterdrückern, Färbemitteln, zusätzlichem Parfüm und Parfümabgabesystemen, Strukturelastisierungsmitteln, Weichspülmitteln, Trägern, Hydrotropen, Verarbeitungshilfsmitteln und/oder Pigmenten und Gemischen davon.

**4.** Verfahren nach Anspruch 3, wobei der Zusatzstoff ein Material umfasst, das ausgewählt aus der Gruppe bestehend aus Färbemitteln; Parfüm; optischen Aufhellern; Abscheidungshilfsmitteln; und Gemischen davon.

## Revendications

**1.** Procédé de production d'une composition de nettoyage comprenant une composition de libération d'agent bénéfique sous la forme d'un agglomérat, d'une matière particulaire ou d'un extrudat et un ingrédient additif, ledit procédé comprenant :

a) la combinaison (i) d'un agent bénéfique encapsulé, dans lequel ledit agent bénéfique encapsulé comprend une microgélule de parfum, ladite microgélule de parfum comprenant une coque, ladite coque comprenant du mélamine-formaldéhyde réticulé (ii) un plastifiant, dans lequel ledit plastifiant comprend de l'eau et iii) un liant, dans lequel ledit liant est choisi dans le groupe constitué de celluloses de préférence la méthylcellulose, plus préférablement la carboxyméthylcellulose, et leurs dérivés ; alginate et ses dérivés ; amidons ; alcools polyvinyliques ; oxyde de polyéthylène ; polyvinylpyrrolidone ; polysaccharides, de préférence le chitosane et/ou des gommes naturelles de préférence le carraghénane ; polyacrylates de préférence des polyacrylates réticulés ; cires ; polyéthylène glycols ayant une masse moléculaire moyenne en poids supérieure à 4000 Da ou même de 4000 Da à 15 000 Da ; éthoxylates d'alcool ; agents tensioactifs et des mélanges de ceux-ci pour former un mélange ;
b) la combinaison dudit mélange avec un agent de poudrage, dans lequel ledit agent de poudrage comprend un matériau choisi dans le groupe constitué de silices ; aluminosilicates de préférence une zéolite ; argiles ; et leurs mélanges pour former un matériau ; et
c) l'élimination d'une quantité suffisante dudit plastifiant dudit matériau pour donner une composition de libération d'agent bénéfique sous la forme d'un agglomérat, d'une matière particulaire ou d'un extrudat comprenant, sur la base du poids total de la composition de libération de 2 % à 97 % en poids d'agent bénéfique encapsulé comprenant une quantité suffisante d'agent bénéfique pour fournir, sur la base du poids total de la composition de libération d'effet bénéfique, de 1 % à 85 % d'agent bénéfique, de 1 % à 50 % en poids, de préférence de 2 % à 45 % en poids de plastifiant, de 1 à 50 % en poids de liant et de 5 % à 50 % en poids d'agent de poudrage ; et
d) le mélange avec un ingrédient additif pour former une composition de nettoyage.

**2.** Procédé selon l'une quelconque revendication précédente, ledit agglomérat, ledit extrudat ou ladite matière particulaire possédant une dimension caractéristique de 100 micromètres à 3000 micromètres, plus préférablement de 200 micromètres à 2500 micromètres, plus préférablement de 300 micromètres à 2000 micromètres, plus préférablement de 400 micromètres à 1400 micromètres, le plus préférablement de 500 micromètres à 1200 micromètres, dans lequel, pour lesdits agglomérats et matières particulaires, ladite dimension caractéristique est la taille médiane de particules desdits agglomérats et matières particulaires et la dimension caractéristique desdits extrudats est le diamètre moyen desdits extrudats.

**3.** Procédé selon l'une quelconque revendication précédente pour fabriquer une composition de nettoyage comprenant une composition de libération d'agent bénéfique selon une quelconque revendication précédente en une quantité, sur la base du poids total de produit de la composition de nettoyage, de 0,1 % à 20 %, et un ingrédient additif, choisi de préférence dans le groupe constitué de polymères, (par exemple, des polymères cationiques), agents tensioactifs, adjuvants, agents chélatants, agents inhibant la décoloration, dispersants, enzymes et agents stabilisant les enzymes, matériaux catalytiques, activateurs de blanchiment, agents de dispersion polymères, agents d'élimination des salissures d'argile/antiredéposition, azurants, suppresseurs de mousse, teintures, parfum et systèmes de libération de parfum supplémentaires, agents d'élastification de structure, adoucissants des tissus, véhicules, hydrotropes, auxiliaires de traitement et/ou pigments et leurs mélanges.

4. Procédé selon la revendication 3, dans lequel l'ingrédient additif comprend un matériau choisi dans le groupe constitué de teintures ; parfum ; azurants optiques ; adjuvants de dépôt ; et leurs mélanges.

**EP 2 247 275 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007099469 A
- WO 2006131846 A
- EP 383289 A
- WO 2006056093 A
- EP 238225 A
- US 6869923 B1
- US 6592990 B2
- US 6544926 B1
- US 4515705 A
- US 4537706 A
- US 4537707 A
- US 4550862 A
- US 4561998 A
- US 4597898 A
- US 4968451 A
- US 5565145 A
- US 5929022 A
- US 6294514 B

- US 6376445 B
- US 5576282 A
- US 6306812 B1
- US 6326348 B1
- US 4430243 A
- US 5597936 A
- US 5595967 A
- WO 0032601 A
- US 6225464 B
- US 5879584 A
- US 5691297 A
- US 5574005 A
- US 5569645 A
- US 5565422 A
- US 5516448 A
- US 5489392 A
- US 5486303 A